# EUROPEAN PATENT SPECIFICATION

(11) **EP 1 728 464 B1**
(45) Date of publication and mention of the grant of the patent: **30.03.2016**
(21) Application number: 06010996.4
(22) Date of filing: 29.05.2006
(51) Int. Cl.: A61B 1/04, A61B 5/00

(54) **Endoscope image pickup system**
Endoskopisches Bildaufnahmesystem
Système endoscopique de prise d'images

(30) Priority: 03.06.2005 JP 2005164807; 07.06.2005 JP 2005167323; 16.06.2005 JP 2005176666
(43) Date of publication of application: 06.12.2006
(73) Proprietor: Olympus Corporation, Shibuya-ku Tokyo (JP)
(72) Inventor: Harano, Kenji, Hachioji-shi Tokyo 192-8512 (JP); Handa, Keiji, Hachioji-shi Tokyo 192-8512 (JP)
(74) Representative: von Hellfeld, Axel

(56) References cited:
- EP-A- 1 488 732
- DE-C1- 19 902 184
- US-B1- 6 212 425
- US-B1- 6 640 131

## Description

### BACKGROUND OF THE INVENTION

### 1. Field of the Invention

The present invention relates to an endoscope image pickup system for fluorescent image pickup using an image pickup device.

### 2. Description of the Related Art

Conventional endoscope systems include those for ordinary endoscopic observation using visible light and those for fluorescent observation by emitting excitation light. And fluorescent observation includes a method called Photodynamic Diagnosis (PDD) in which a fluorescent substance is accumulated only in a tumor portion by administration of a pharmaceutical agent and making a diagnosis by emitting excitation light to have the tumor emit fluorescence.

PDD emits fluorescence when blue excitation light is applied. Therefore, by arranging a band-pass filter on a light source apparatus side as an excitation light transmission filter transmitting blue color so that blue excitation light is emitted, the light transmitted by this band-pass filter is incident into a light guide of an endoscope.

In this case, the fluorescence is weaker than the excitation light. Therefore, in order to obtain a fluorescent image, it is necessary to cut the excitation light on the light receiving side and to transmit the fluorescence, and a cut filter is used.

However, if the excitation light is completely cut by the cut filter, a living state other than the fluorescent part becomes invisible.

Therefore, it is necessary for the light receiving side to create a blue illumination light for background so that a certain level of excitation light is transmitted while balancing with the fluorescence. Thus, the cut filter transmits a part of the blue illumination light as excitation light.

If the transmission light intensity with respect to the blue illumination light is not constant, a hue contrast against the fluorescent part becomes poor, which affects determination of a tumor. However, a wavelength of the filter has large variations, and there is a problem that it is difficult to make the illumination light for the background formed by an overlapping part of light transmission by combination of two filters constant.

Then, as disclosed in German Patent No. DE19902184, a wavelength of an outgoing light is made constant by adjusting an angle of a band-pass filter (excitation light transmission filter) on the light source apparatus side.

However, in German Patent No. DE19902184, when a plurality of endoscopes are used for a single light source apparatus, cut filters have variations in characteristics for each of the endoscopes, and when used in combination with the endoscopes, angles of the excitation light transmission filters on the light source apparatus need adjustment for each of the endoscopes.

Particularly, when an endoscope image pickup system is constituted by attaching an image pickup apparatus such as a TV camera to an endoscope, it is also necessary to adjust an angle of the excitation light transmission filter on the light source apparatus side for each of the endoscopes, which has a room for further improvement.

Also, with a construction as in Germany Patent No. DE19902184 that an angle of the excitation light transmission filter is adjusted on the light source apparatus side, if a plurality of endoscopes are used for a single light source apparatus, cut filters have variations in characteristics for each of the endoscopes, and the angle of the excitation light transmission filter needs to be adjusted on the light source apparatus side for each of the endoscopes when used in combination with the endoscopes.

In this case, since an operator makes fluorescent pattern. The first lymphatic node reached a cancer cell having entered into the lymphatic vessel from a primary focus of the cancer is called as Sentinel Lymph Node. When the cancer has metastasized to the lymphatic node, it is considered that there is metastasis in the sentinel lymph node. Therefore, presence of metastasis to a lymphatic node can be determined by discovering a sentinel lymph node during a cancer ablative operation for an early stage cancer, performing a biopsy and rapidly conducting a pathological inspection.

As a conventional detecting method of a sentinel lymph node, a dye method using a blue dye is known. A blue dye such as patent blue or phazurin is locally injected around the cancer percutaneously or using an endoscope immediately before a cancer ablation surgery. The injected dye moves from the injected portion to a lymphatic vessel and reaches a sentinel lymph node in 5 to 15 minutes.

An observer visually detects a blue-dyed sentinel lymph node. However, lymphatic nodes are often covered by living tissues such as fat, and it is necessary to search a sentinel lymph node while separating the living tissues. Thus, it takes time till detection and the dye might reach a lymphatic node at the downstream of the sentinel lymph node during that period, which causes a problem that detection of the sentinel lymph node becomes difficult.

Also, an RI method using a radioisotope as a tracer has been developed and is being put into practical use. With the RI method, a radioisotope is locally injected around a cancer percutaneously or using an endoscope on a day prior to an operation. The injected radioisotope moves to a lymphatic vessel from the injected portion and remains in a sentinel lymph node for a certain period of time. Lymphoscintigraphy is performed several hours after injection of the radioisotope and a position is roughly marked. Then, the position is ablated in an ablative operation of a cancer, a gamma ray dose radiated from a lymphatic node around the ablated position is detected using a gamma probe, and the lymphatic node radiating the highest gamma ray dose is detected as a sentinel lymph node.

With the RI method, since the tracer reaches a downstream lymphatic node in a short time, detection is not difficult. Also, since a sentinel lymph node covered by a living tissue can be detected, a detection rate is improved. However, since a radiation ray is used, operation management is complicated. And since a gamma ray dose is detected by a gamma probe, it is necessary to detect one by one, which makes image formation impossible. Moreover, there is a problem both for the dye method and the RI method that presence of metastasis should be finally determined by ablating a sentinel lymph node and conducting a pathological diagnosis during or after the operation.

In order to solve the above problems, a sentinel lymph node detecting system using a fluorescent substance method is proposed as Japanese Patent Laid-Open No. 2001-299676 in which a light-sensitive substance with an affinity to a tumor and emitting fluorescence when excited by light is administered as a fluorescent diagnostic agent in a living body in advance, fluorescence is generated from the fluorescent diagnostic agent accumulated in a sentinel lymph node by emitting excitation light in an excitation wavelength band of the light-sensitive substance and the sentinel lymph node is detected by receiving the fluorescence.

However, in this proposal, the wavelength band of the emitted excitation light is substantially equal to the wavelength band of the fluorescence, and it is difficult to separate the fluorescence generated from the sentinel lymph node from reflected light of the excitation light. Thus, there is a problem that the detection sensitivity of the fluorescence is lowered and the detection accuracy of the sentinel lymph node is deteriorated.

In view of the above-mentioned problems, the present invention has an object to provide an endoscope image pickup system which enables fluorescent observation in an appropriate hue contrast even if filter characteristics have variations, without requiring angle adjustment of an excitation light transmission filter on the light source apparatus side.

Moreover, in view of the above-mentioned problems, the present invention has an object to provide an endoscope image pickup system which enables fluorescent observation in an appropriate hue contrast with good workability even if filter characteristics have variations, without requiring angle adjustment of an excitation light transmission filter on the light source apparatus side.

Furthermore, in view of the above-mentioned problems, the present invention has an object to provide an endoscope image pickup system which can detect a sentinel lymph node in a short time with a high accuracy.

United States Patent No. 6,212,425 describes an example of an endoscope apparatus in which also part of the light from the excitation range reaches the image plane of the image-generating unit of the device.

### SUMMARY OF THE INVENTION

The present invention provides an endoscope image pickup system as defined in claim 1.Preferred features of the invention are recited in the dependent claims.

Accordingly, an endoscope image pickup system of the present invention comprises light source means for generating light in a visible light wavelength region including blue excitation light;
an excitation light transmission filter provided at the light source means and transmitting the blue excitation light;
an ordinary light filter provided at the light source means for transmitting light in the visible light wavelength region;
an endoscope for irradiating light from the light source means so as to obtain an optical subject image using light reflected by a subject;
an endoscopic filter provided at the endoscope for transmitting only a part of the light in the visible light wavelength region and of the excitation light and transmitting fluorescence;
image pickup means attached to an ocular portion of the endoscope for picking up an image of the subject according to each of the lights transmitted by the endoscopic filter; and
image processing means for processing an image pickup signal from the image pickup means for image formation,
wherein the excitation light transmission filter and the ordinary light filter are switchable such that the excitation light transmission filter is arranged on the optical path of the light source means when the system is in a fluorescent observation mode and the ordinary light filter is arranged on the optical path of the light source means when the system is in an ordinary observation mode,
wherein the endoscope image pickup system includes:
composite image generating means provided at the image processing means, and
color tone correcting means,
wherein the color tone correcting means is adapted to obtain a gain value for adjusting a blue color signal outputted from a signal processing circuit connected to the image pickup means to a constant value when an image of an inner surface of a B balance cap irradiated with the blue excitation light is picked up, and correct a luminance level of a blue excitation light image obtained by picking up an image of the subject by using the obtained gain value, and the composite image generating means is adapted to generate a composite image by combining a fluorescent image obtained by picking up an image of the subject and a blue excitation light image corrected by the color tone correcting means, when the system is in the fluorescent observation mode, and
the composite image generating means is adapted to generate a composite image from different color signals corresponding to component images obtained using the light in the visible light wavelength region and the color tone correcting means is adapted to amplify the color signal corresponding to the wavelength of the excitation light, when the system is in the ordinary observation mode.

### BRIEF DESCRIPTION OF THE DRAWINGS

Fig. 1 is a schematic block diagram of an entire construction of an endoscope image pickup system of a first preferred embodiment;
Fig. 2 is a block diagram showing an inside construction of a camera control unit;
Fig. 3 is a characteristic graph of spectral transmission characteristics of a filter provided at a light source apparatus and a filter provided at an endoscope;
Fig. 4 is a view showing a state where a tip end side of the endoscope is inserted into a B balance cap;
Fig. 5 is a characteristic graph of spectral transmission characteristics of a filter provided at a light source apparatus and a filter provided at an endoscope in a second embodiment which is not forming part of the invention;
Fig. 6 is a block diagram showing an internal construction of CCU in a variation of the second embodiment;
Fig. 7 is a view showing a construction of an endoscope image pickup system of a third embodiment which is not forming part of the invention;
Fig. 8 is a view showing a construction of an ocular portion of the endoscope;
Fig. 9 is a characteristic graph of spectral transmission characteristics of a band-pass filter arranged in a light source apparatus and a cut filter provided at an endoscope for cutting excitation light;
Fig. 10 is a view showing a construction of the vicinity of an ocular portion in a variation of the third embodiment;
Fig. 11 is a sectional view showing a schematic construction of the vicinity of an ocular portion of an endoscope in a fourth embodiment which is not forming part of the invention;
Fig. 12 is a perspective view showing a structure of an eye piece for fluorescence of the fourth embodiment,;
Fig. 13 is a sectional view showing a schematic structure of the vicinity of the ocular portion of the endoscope in a first variation of the fourth embodiment;
Fig. 14 is a perspective view showing a structure of an eye piece for fluorescence in a second variation of the fourth embodiment;
Fig. 15 is a block diagram for explaining, an entire construction of an endoscope image pickup system according to a fifth embodiment which is not forming part of the invention;
Fig. 16 is a graph for explaining an example of transmission characteristics of a filter and a cut-off filter used in a fluorescent observation mode in the fifth embodiment;
Fig. 17 is a schematic view for explaining arrangement of a scope at inspection; and
Fig. 18 is a graph for explaining an example of transmission characteristics of a filter and a cut-off filter used in a fluorescent observation mode in a sixth embodiment which is not forming part of the invention.

### DESCRIPTION OF THE PREFERRED EMBODIMENTS

Embodiments 2 - 6 do not form part of the present invention.

### (First preferred embodiment)

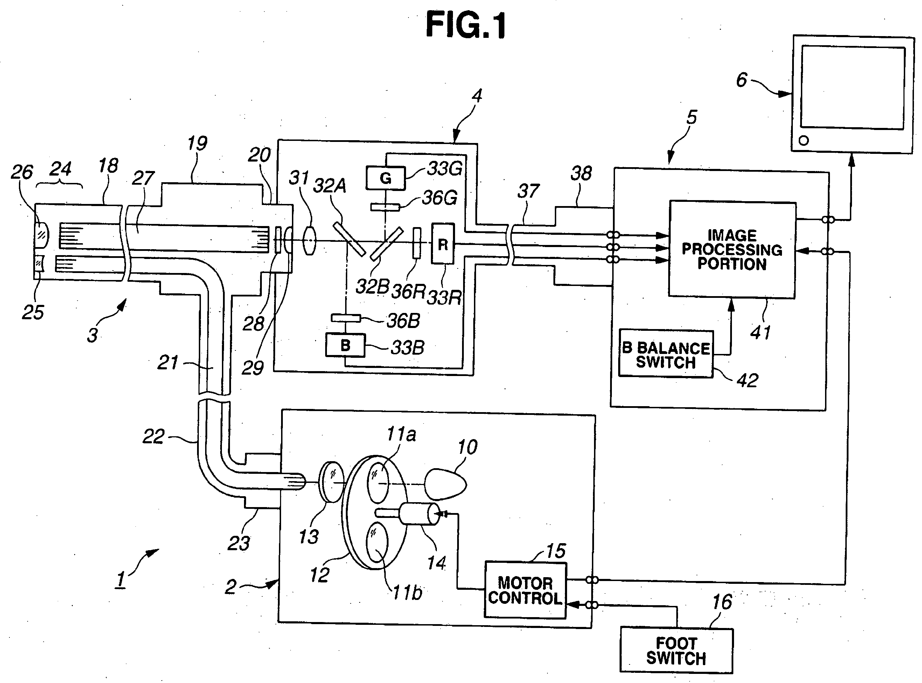

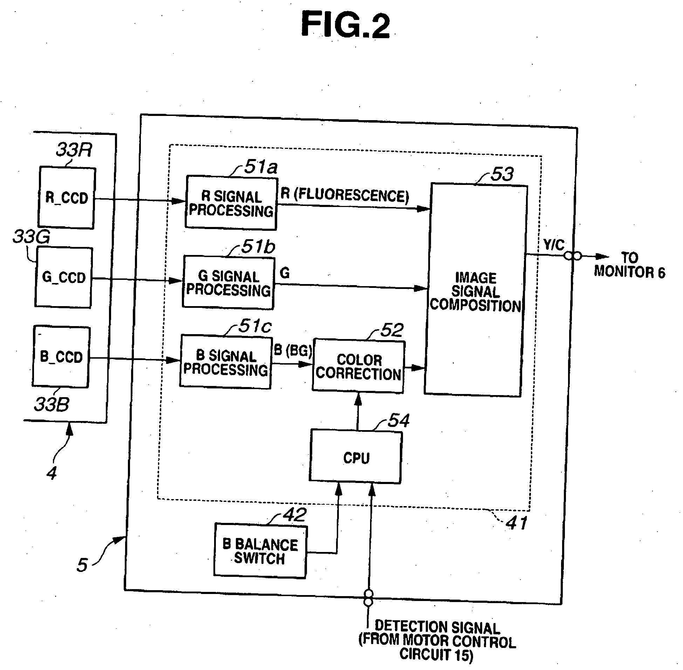

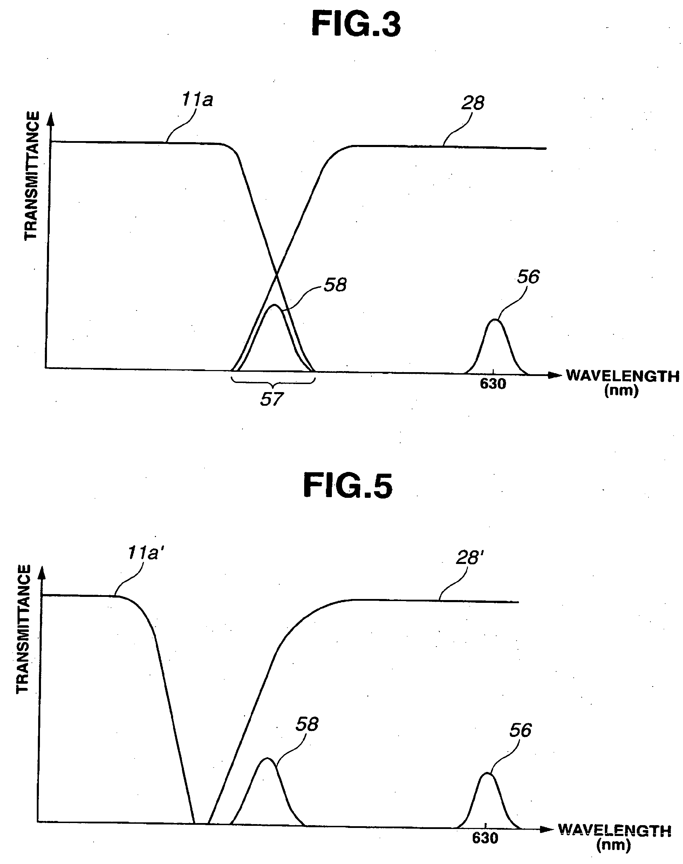

Figs. 1 to 4 are views according to a first preferred embodiment. Fig. 1 is a schematic block diagram of an entire construction of an endoscope image pickup system Fig. 2 is a block diagram showing an inside construction of a camera control unit, Fig. 3 is a characteristic graph of spectral transmission characteristics of a filter provided at a light source apparatus and a filter provided at an endoscope and Fig. 4 is a view showing a state where a tip end side of the endoscope is inserted into a B balance cap.

First, the construction of the first preferred embodiment will be described. As shown in Fig. 1, an endoscope image pickup system 1 of the first preferred embodiment of the present invention comprises a light source apparatus 2 for generating an illumination light, an optical endoscope 3 to be inserted into a body cavity for observing a subject image, a camera head 4 detachably connected to an ocular portion 20 of this endoscope 3, a camera control unit (hereinafter referred to as CCU) 5 for performing image processing to create an image from an image pickup signal by an image pickup device in this camera head 4 and outputting a standard image signal, and a monitor 6 for displaying the image signal outputted from this CCU 5.

The light source apparatus 2 is provided with a lamp 10 such as a xenon lamp for emitting light, a turret 12 provided on an illumination optical path of this lamp 10 and provided with a blue light filter 11a for transmitting blue excitation light as an excitation light transmission filter for transmitting excitation light and an ordinary light filter 11b for transmitting light in an ordinary visible region, a condenser lens 13 for condensing light transmitted by the filter 11a or the filter 11b, a motor 14 for rotating the turret 12 and a motor control circuit 15 or the like for controlling rotation of the motor 14.

This motor control circuit 15 is connected to an external foot switch 16 or the like, and an operator operates this foot switch 16 so as to instruct switching of an observation mode, and a signal of the switching instruction is inputted into the motor control circuit 15. Receiving this signal, the motor control circuit 15 switches the filter arranged on the optical path by rotating the motor 14.

when switching to an ordinary observation is instructed, the ordinary light filter 11b is arranged on the optical path and set in the illumination state for the ordinary observation mode, while when switching to a fluorescent observation is instructed, the blue light filter 11a is arranged on the optical path and set in the illumination state for the fluorescent observation mode. And light having passed the filter arranged on the optical path is collected and is incident on a light guide of the endoscope 3.

The endoscope 3 has an elongated insertion portion 18 to be inserted into a body cavity, an operation portion 19 provided at its rear end and ocular portion 20 provided at a rear end of this operation portion 19.

Also, a light guide 21 for transmitting an illumination light is inserted through the insertion portion 18, this light guide 21 being inserted into a light guide cable 22 extended from the operation portion 19, and a light guide connector 23 at its rear end is detachably connected to the light source apparatus 2. And the illumination light from the light source apparatus 2 is incident on an incident end face of this light guide connector 23.

This incident illumination light is transmitted to a tip end face of the light guide 21. The tip end face of this light guide 21 is fixed to an inside of an illumination window provided at a tip end portion 24 of the insertion portion 18, and the illumination light outputted from the tip end face is emitted to a subject side such as an affected area in the body cavity through an illumination lens 25 mounted at this illumination window.

An objective lens 26 is mounted at an observation window provided adjacent to the illumination window, and a subject image is formed by returned light from the subject (reflected light in the case of the ordinary observation and reflected light and fluorescence in the case of the fluorescent observation) on a tip end face of an image guide 27 arranged at an image forming position by this objective lens 26.

The optical image on the tip end face is transmitted to a rear end face by this image guide 27. This rear end face is arranged in the vicinity of the ocular portion 20, and opposite to the rear end face, a cut filter 28 for cutting a part of a long wavelength side of blue light to become excitation light and the ocular lens 29 are arranged. Transmission wavelength characteristics of this cut filter 28 and the blue light filter 11a for transmitting blue excitation light in the light source apparatus 2 are shown in Fig. 3.

In the camera head 4 detachably attached to this ocular portion 20, an image forming lens 31 for forming an image of the subject transmitted to the ocular portion 20 of the endoscope 3 on the image pickup device is arranged opposite to the optical axis of the ocular lens 29.

At a middle position on the optical axis to the image forming position of this image forming lens 31, a dichroic mirror 32A and a dichroic mirror 32B for selectively reflecting or transmitting light in a specific wavelength region are arranged sequentially.

The dichroic mirror 32A has a characteristic to reflect light with blue wavelength and to transmit the other visible light components. Also, the dichroic mirror 32B has a characteristic to reflect a green light component in the visible light and to transmit the remaining visible light components, which is red light in this case.

Therefore, an optical image of the blue light component is formed at an image forming position on the side reflected by the dichroic mirror 32A, a green optical image is formed at an image forming position on the side transmitted by this dichroic mirror 32A and reflected by the dichroic mirror 32B, and a red light optical image is formed at an image forming position on the side transmitted by the two dichroic mirrors 32A and 32B.

And at the image forming position on the side reflected by the dichroic mirror 32A, B_CCD 33B for capturing an image of the blue light component is arranged.

A B band-pass filter 36B for transmitting the blue light component and limiting transmission of light other than this blue light component is arranged on the optical axis between the dichroic mirror 32A and the B_CCD 33B.

Also, a G_CCD 33G for capturing an image of a green light component is arranged at the image forming position on the side transmitted by the dichroic mirror 32A and reflected by the dichroic mirror 32B. Moreover, a G band-pass filter 36G for transmitting the green light component and limiting transmission of light other than this green light component is arranged on the optical axis between the dichroic mirror 32B and the G_CCD 33G.

Moreover, an R_CCD 33R for capturing an image of a red light component is arranged at the image forming position on the side transmitted both by the dichroic mirrors 32A and 32B, and an R band-pass filter 36R for limiting light other than the red light is arranged on the optical axis between the dichroic mirror 32B and the R_CCD 33R.

Each of the optical images received by each of the B_CCD 33B, G_CCD 33G and R_CCD 33R is converted to an electric signal to become an image pickup signal. Signal line whose one ends are connected to each of B_CCD 33B, G_CCD 33G and R_CCD 33R, respectievly, are inserted through a camera cable 37 extended from the camera head 4 and connected to a contact of a signal connector 38. And this signal connector 38 is detachably connected to the CCU 5.

The CCU 5 has an image processing portion 41 and a B balance switch 42, and the image pickup signal transmitted from the camera head 4 is image-processed at the image processing portion 41 so as to generate an image signal, which is outputted to the monitor 6 so as to display an ordinary image and a fluorescent image.

Fig. 2 shows an internal construction of the CCU 5 for performing image processing of the image pickup signal in this preferred embodiment.

As shown in Fig. 2, the image processing portion 41 in the CCU 5 comprises R, G, B signal processing circuits 51a to 51c connected to the CCD 33R, 33G, 33B of R, G, B, respectively, a color correction circuit (or brightness correction circuit) 52 connected to the B signal processing circuit 51c, an image signal composite circuit (image signal composite circuit) 53 for composing R, G, B signals, and a CPU 54 for controlling the color correction circuit 52 and the image signal composite circuit 53.

To the R, G, B signal processing circuits 51a to 51c, an image pickup signal image-captured by the CCD 33R, 33G, 33B of each of R, G, B is inputted, and only a signal component of each pixel is extracted by correlated double sampling processing (CDS processing) or the like so as to generate color signals R, G, B. That is, the R, G, B signal processing circuits 51a to 51c perform signal processing of image pickup signals which are image-captured by the CCD 33R, 33G, 33B for image capturing by separating an optical image transmitted to the ocular portion 20 of the endoscope 3 into wavelength regions so as to generate the color signals R, G, B corresponding to component images of each of the wavelength regions.

In this case, in the ordinary observation mode, the R, G, B signal processing circuits 51a to 51c generate the color signals R, G, B in this way, while in the fluorescent observation mode, the R, B signal processing circuits 51a, 51c generate a fluorescent image and an excitation light image (blue image) described as an illumination light 58 for background in Fig. 3 obtained using a blue excitation light.

And output signals of the R, G, B signal processing circuits 51a to 51c are combined by the image signal composite circuit 53 for composing a color image for color display so as to generate a luminance signal Y and a color signal C corresponding to a composite image, which are outputted to the monitor 6.

Here, only the B signal processing circuit 51c has a luminance level of the color signal B corrected at the color correction circuit 52 provided between the B signal processing circuit 51c and the image signal composite circuit 53 according to control from the CPU 54 in the case of the fluorescent observation mode. And the fluorescent image obtained using fluorescence and the excitation light image obtained using the excitation light are contrast-corrected by the image signal composite circuit 53 for the case of color composition for color display so that color image composition is performed with an appropriate hue contrast.

In the case of the ordinary observation mode as above, the R, G, B signal processing circuits 51a to 51c perform signal processing for the output signals of the CCD 33R, 33G, 33B capturing color components of R, G, B and output the color signals of R, G, B, respectively.

Moreover, in the case of the fluorescent observation mode, the R signal processing circuit 51a outputs an image signal of a fluorescent image and the B signal processing circuit 51c outputs an image signal (abbreviated as BG in Fig. 2) corresponding to an excitation light image captured by the illumination light 58 for background on the long wavelength side of the blue excitation light. If fluorescence is also generated in the green wavelength region other than the red wavelength, the G signal processing circuit 51b also outputs an image signal of the fluorescent image.

The color correction circuit 52 is set, in the case of fluorescent observation by emitting blue light as the excitation light, so that a blue set value as setting information to set an appropriate hue contrast between a fluorescent image obtained using fluorescence in the red wavelength region and a blue excitation light image is inputted in advance (which will be described later).

The CPU 54 is connected to the motor control circuit 15 of the light source apparatus 2, and a detection signal is inputted indicating whether the current illumination light is the blue light filter 11a or the ordinary light filter 11b. By this, the CPU 54 is constituted to control the color correction circuit 52 according to determination that the current illumination light state is the ordinary light observation state (ordinary observation mode) or the fluorescent observation state (fluorescent observation mode)

Fig. 3 shows transmission wavelength characteristics of the blue light filter 11a of the light source apparatus 2 and the cut filter 28 provided in the ocular portion 20 of the endoscope 3. When a blue excitation light having passed the blue light filter 11a is emitted from the tip end of the endoscope 3, only a tumor portion containing a fluorescent agent as a light-sensitive substance becomes fluorescence 56 whose intensity is peaked in a red wavelength region or specifically in the vicinity of 630 nm, for example, as shown in Fig. 3, which is image-captured by the R_CCD 33R.

However, since a red fluorescence is smaller than the blue light, a part of the blue light is cut by the cut filter 28. However, if all the blue light is cut off, it makes a fluorescent image with a portion other than the fluorescent portion generating fluorescence invisible. Thus the blue illumination light 58 for background is created by providing an overlap portion 57 where transmission characteristics of both the filters overlap to some extent, and a blue excitation light image is generated by this illumination light 58.

If a proportion of a transmission portion of the illumination light 58 generating this blue excitation light image is large, red fluorescence is weakened and a hue contrast becomes poor. or, if a proportion of a transmission portion of the illumination light 58 generating this blue excitation light image is small, the fluorescence (green to red light) side of a living tissue by the excitation light becomes dominant, which whitens the tissue and similarly deteriorates the hue contrast.

Thus, it is preferable that an intensity of the blue illumination light 58 for background is appropriate, but both the blue light filter 11a and the cut filter 28 have variations within wavelength tolerances and a light intensity of the illumination light 58 for background is fluctuated by combination of the two filters. Thus, in this preferred embodiment, the fluctuation is suppressed as below by image processing performed in the CCU 5 as image processing means.

Fig. 4 shows a B balance cap 61 used when using the B balance switch 42 of the CCU 5. This B balance cap 61 is in a cylindrical shape with a bottom, for example, and its inside is coated by a paint proximate to a reflected wavelength characteristic, which is a reflected light intensity suitable for fluorescent observation when blue light is emitted to a living tissue in advance.

And color correction is made by the color correction circuit 52 so that an output signal of the B signal processing circuit 51c which picked up an image in this case becomes a constant value. By this, even if the light intensity of the illumination light 58 is fluctuated by combination of the filters, a color composite image capable of fluorescent observation in an appropriate hue contrast can be generated without requiring control work of transmission characteristics by means of angle adjustment on the light source apparatus 2 side with respect to the blue light filter 11a.

Next, operation of the first preferred embodiment will be described.

When the state as shown in Fig. 1 is set and power is turned on, the motor control circuit 15 of the light source apparatus 2 sets arrangement of the ordinary light filter 11b on an illumination optical path as an initial state, for example.

And a white subject is prepared by using a cap for white balance, not shown, or the like, and a white balance switch is turned on. Then, the CPU 54 sets a gain of an internal amplifier so that the output signals of two signal processing circuits of the R, G, B signal processing circuits 51a to 51c (here, the G, B signal processing circuits 51b, 51c) match the luminance level of an output signal of the remaining R signal processing circuit 51a.

That is, the white balance state is set so that a white subject is displayed in white. In this case, the color correction circuit 52 lets the input signal go through, for example, and outputs it (or changes the gain of the internal amplifier of the color correction circuit 52 to 1).

In the first preferred embodiment, since the cut filter 28 is arranged at the ocular portion 20 of the endoscope 3, the blue short wavelength side is cut off, and a blue component is smaller than green and red incident light when image-captured. But by setting the gain of the amplifier larger than those of the other circuits at the B signal processing circuit 51c in the above-mentioned white balance processing, the white balance state is ensured and ordinary observation can be made.

After completion of this white balance control, an operator operates the foot switch 16 and makes switching to arrange the blue light filter 11a on the illumination optical path. In this case, blue light is emitted from the tip end of the endoscope 3.

Here, the operator places the tip end of the endoscope 3 into the B balance cap 61 as shown in Fig. 4, presses the B balance switch 42 of the CCU 5 and conducts adjustment processing so that the observation state in the fluorescent observation mode can be made (displayed) with an appropriate hue contrast.

When the tip end of the endoscope 3 is inserted into the B balance cap 61 as mentioned above and the B balance switch 42 is pressed, blue reflected light reflected by an inner surface of the B balance cap 61 is received by the B_CCD 33B, and its color signal B is created at the B signal processing circuit 51c.

At this time, based on control of the CPU 54, the color correction circuit 52 adjusts contrasting density of a blue paint or the gain of the amplifier in the color correction circuit 52 so that the measured blue color signal B becomes an appropriate blue set value and stores it as a correction value (gain value) in memory means such as a memory in the color correction circuit 52.

If the fluorescent observation is to be made after that, this blue correction value is reflected in the color correction circuit 52 (the above amplifier gain value is maintained), and variations in color tone generated by a difference in light intensity of the illumination light 58 (generating an excitation light image) caused by combination of filters ca be suppressed.

When the fluorescent observation is to be actually made for a living tissue, a color signal corresponding to a fluorescent image from the R signal processing circuit 51a and a signal corresponding to an excitation light image from the color correction circuit 52 are made color-composite by the image signal composite circuit 53, and identification display (e.g.: superimpose) of the color-composite fluorescent image is superimposed on the color fluorescent image by control from the CPU 54 and outputted to the monitor 6. By this, the operator can observe the color fluorescent image with the appropriate hue contrast.

Therefore, the first preferred embodiment has the following effects.

According to the construction of the above-mentioned first preferred embodiment, since the variations by filter combination can be suppressed by image processing, a fluorescent image with an appropriate hue contrast can be obtained all the time. By this, even an extremely small tumor can be easily determined, and the operator can perform inspection or procedures such as tumor ablation with efficiency.

Also, since endoscopic inspections are normally conducted using a plurality of endoscopes 3 and camera heads 4 to a single light source apparatus 2 in a hospital, this case can be appropriately coped with. That is, color correction corresponding to combination with the cut filter 28 of the endoscope 3 can be easily performed on the CCU 5 side as the image processing device without requiring adjustment of transmission characteristics of the blue light filter 11a as an excitation light transmission filter on the light source apparatus 2 side.

Moreover, if an adjustment amount of blue paint is made changeable, a hue preferred by the operator can be set.

It is to be noted that in the first preferred embodiment, a camera head adopting a three-plate type image pickup device in which a color is separated into three color components and an optical image of each of the components is captured individually is used as the camera head 4 attached to the ocular portion 20 of the optical endoscope 3, but similar application can be realized in the case of a camera head using a single-plate type image pickup device in which a color filter such as a mosaic filter is mounted on an image pickup surface.

When the single-plate type image pickup device is used, a color separation circuit is provided on the CCU 5 side, and for a signal after separation into a color signal, color correction or brightness correction may be conducted for a signal corresponding to an excitation light image as shown in Fig. 2 and combined with a signal of a fluorescent image.

Also, a variation of the first preferred embodiment may be an electronic endoscope provided with an image pickup device such as the optical endoscope 3 and the camera head 4. In this case, it is only necessary that the cut filter 28 is arranged in front of the image pickup surface of the image pickup device. Also, the image pickup device may be a type for capturing an optical image transmitted by an image guide 27 or have a construction arranged at an image forming position of the objective lens 26.

### (Second embodiment no part of the present invention)

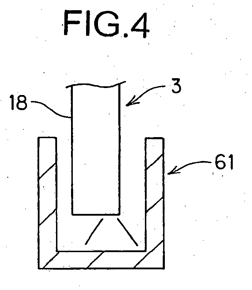

Next, a second embodiment will be described referring to Fig. 5. The construction of the second embodiment is substantially the same as that of the first preferred embodiment, but the light source apparatus 2 and the endoscope 3 adopt filters having different transmission characteristics.

In the second embodiment 2, as shown in Fig. 5, a double band-pass filter is adopted in which an excitation light transmission filter 11a' of the light source apparatus 2 has a transmission range of the blue light filter 11a with blue excitation light and a transmission range of an illumination light 58 for background set on the long wavelength side so that it does not overlap the transmission range of the blue excitation light.

Also, this illumination light 58 for background is set in the transmission wavelength of a cut filter 28' of the endoscope 3.

The transmission range generating the illumination light 58 in the excitation light transmission filter 11a' has a wavelength tolerance set to fall within the transmission wavelength of the cut filter 28' even if there is a variation due to this filter 11a'.

The other construction is the same as that of the first preferred embodiment. Next, operation of the second preferred embodiment will be described.

A basic portion of the operation of the second preferred embodiment is the same as that of the first preferred embodiment, but by construction with the filter as shown in Fig. 5, the variation of the illumination light 58 depends only on the excitation light transmission filter 11a' of the light source apparatus 2, and the variation can be suppressed.

Also, since the variation of the illumination light 58 depends only on the excitation light transmission filter 11a' on the light source apparatus 2 side, once blue correction is performed by operating the B balance switch 42, no further correction is necessary even if the endoscope 3 is changed, which is an advantage.

Therefore, the second preferred embodiment has the following effect.

With the above construction, once the blue correction is performed by operating the B balance switch 42 for the filter characteristic of the excitation light transmission filter 11a' of the light source apparatus 2, correction at each switching of the endoscope 3 is not needed any more.

Thus, if blue correction is performed at a shipment inspection of the light source apparatus 2, it is not necessary for a user to conduct the blue correction any more and time for endoscope inspections, operations or the like using the endoscope 3 can be reduced.

Also, it is not necessary to enclose the B balance cap 61 in the endoscope image pickup system 1.

Though the light in the blue wavelength region is used as the excitation light, an image is captured by fluorescence in the red wavelength region and blue illumination light is used for background in the above description, it is possible to constitute so that the illumination light for background can handle any wavelength region ranging from green to blue, for example.

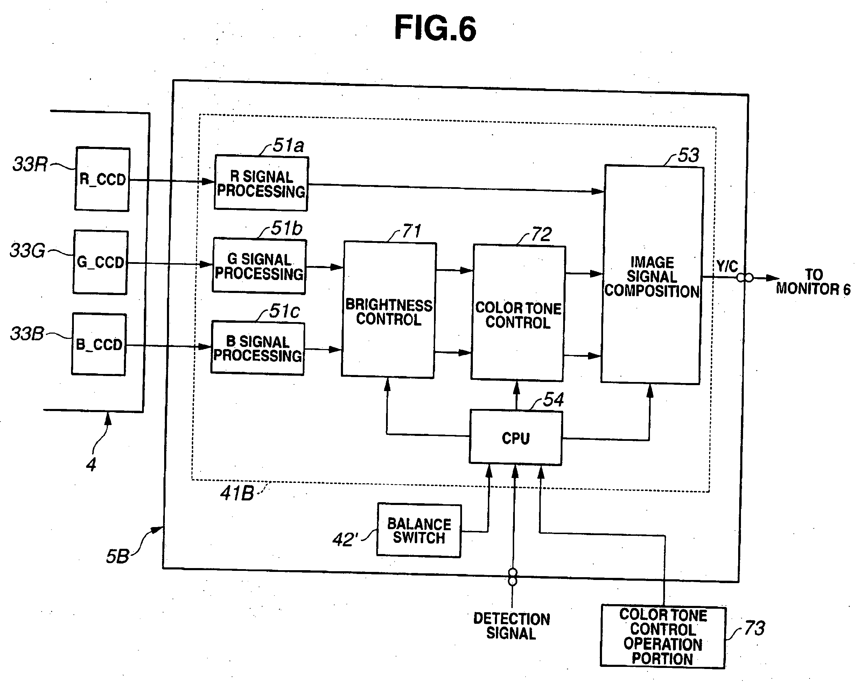

Fig. 6 shows a construction of an image processing portion 41B of CCU 5B in a variation of the second preferred embodiment corresponding this case. In the variation of the second preferred embodiment, the image processing portion 41 described in the first preferred embodiment is constituted, as shown in Fig. 6, such that output signals of the G signal processing circuit 51b and the B signal processing circuit 51c are inputted to the image signal composite circuit 53 through a brightness control circuit 71 for adjusting the luminance level of both the signals and a color tone control circuit 72 for changing hue or color tone from both the signals.

Also, in the variation of the second preferred embodiment, a balance switch 42' is adopted instead of the B balance switch 42. In this case, a balance cap is used instead of the B balance cap 61, and by using this balance cap, an appropriate value of signal intensity on the excitation light image side can be set in the brightness control circuit 71 in case of the color composite of a fluorescent image and an excitation light image.

The CPU 54 is connected to a the color tone control operation portion 73, and when an operator operates a variable resistance constituting the color tone control operation portion 73, for example, the CPU 54 changes the hue or color tone when G and B are to be combined by the color tone control circuit 72. The other construction is the same as that of the second preferred embodiment.

In this variation, in the fluorescent observation mode, a signal of a fluorescent image is inputted from the R signal processing circuit 51a to the image signal composite circuit 53, and in this case, a signal corresponding to the excitation light image captured with excitation light is inputted to the image signal composite circuit 53 from either or both of the G signal processing circuit 51b and the B signal processing circuit 51c.

And by this image signal composite circuit 53, color composite is effected from the signal of the fluorescent image and the signal of the excitation light image. The operator can set hue or color tone preferred by the operator and make fluorescent observation by operating the color tone control operation portion 73.

The image signal composite circuit 53 is not limited to those for outputting an image signal converted from a color signal to a luminance signal and a color difference signal but can be constituted so that the color signal as it is or after matrix conversion is applied to the R, G, B channels of the monitor 6.

### (Third embodiment not part of the present invention)

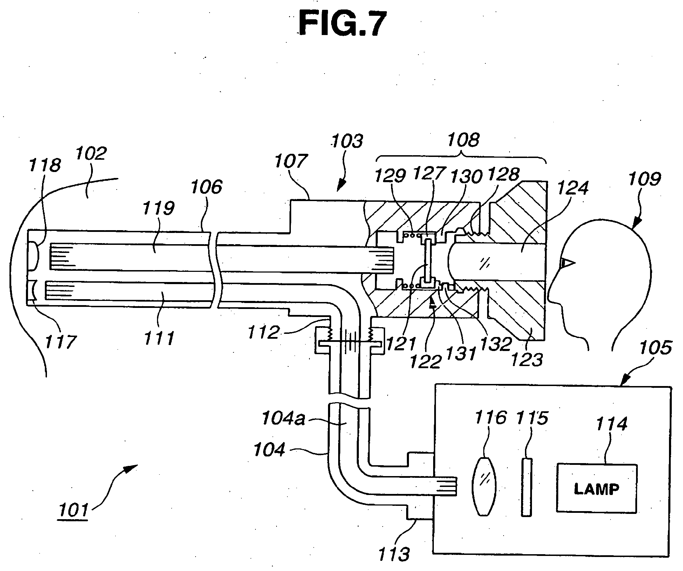

Next, a third embodiment will be described below referring to Figs. 7 to 10.

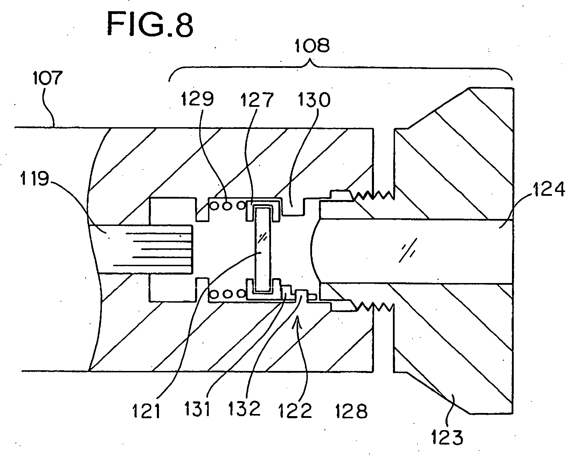

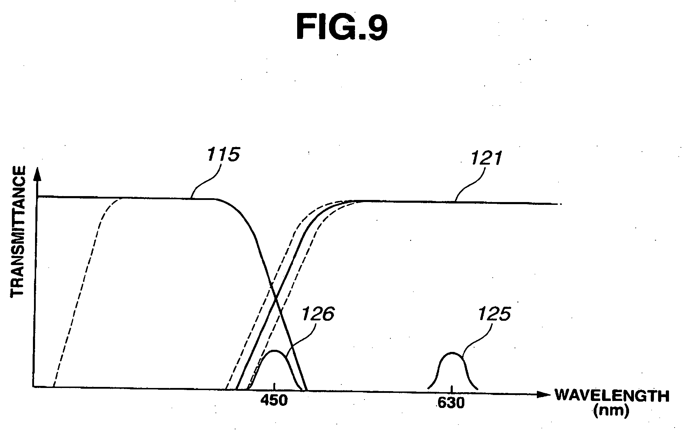

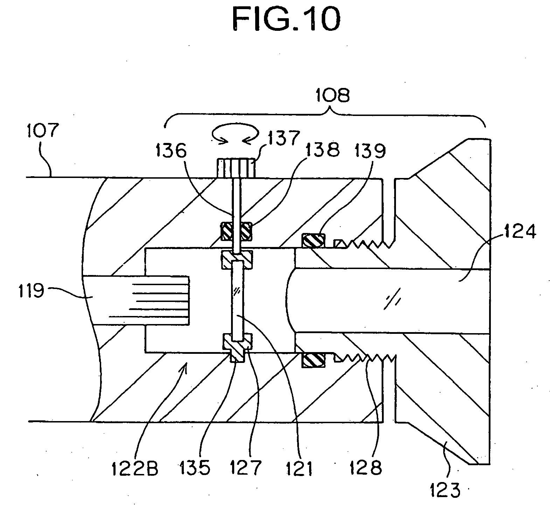

Figs. 7 to 10 are views according to the third embodiment. Fig. 7 is a view showing the construction of an endoscope system of the third preferred embodiment of the present invention. Fig. 8 is a view showing the construction of an ocular portion of the endoscope. Fig. 9 is a graph showing spectral transmission characteristics of a band-pass filter arranged in a light source apparatus and a cut filter provided at the endoscope for cutting excitation light. Fig. 10 is a view showing the construction of the vicinity of the ocular portion in a variation.

First, the construction of this preferred embodiment will be described. As shown in Fig. 7, an endoscope system 101 as an endoscope image pickup system in the third preferred embodiment has an endoscope 103 to be inserted into a body cavity 102 for conducting endosopic observation or inspections of a subject such as an affected area in the body cavity and a light source apparatus 105 for supplying an illumination light from blue light to visible light to the endoscope 103 through a light guide cable 104.

The endoscope 103 has an elongated insertion portion 106 to be inserted into the body cavity, an operation portion (or gripping portion) 107 provided at a rear end of this insertion portion 106 and gripped by an operator 109 and an ocular portion 108 provided at a rear end of this operation portion 107 and visually observed by the operator 109.

Also, a light guide 111 for transmitting an illumination light is inserted into the insertion portion 106, and this light guide 111 is connected to the light guide cable 104 into which a light guide 104a is inserted at a light guide base portion 112 of the operation portion 107, and a light guide connector 113 at the rear end of this light guide cable 104 is detachably connected to the light source apparatus 105.

In the light source apparatus 105, a lamp 114 such as a xenon lamp generating illumination light of substantially white light covering the wavelength region of a visible light is provided. And the illumination light generated by this lamp 114 is incident into an excitation light transmission filter 115 made of a high-pass filter or a band-pass filter capable of angle adjustment provided on its illumination optical path for transmitting the PDD excited wavelength and cutting off a long wavelength side.

The light transmitted by this excitation light transmission filter 115 is incident on an end face of the light guide 104a of the light guide connector 113 through a condenser lens 116 for condensing the illumination light.

The spectral transmission characteristic of the excitation light transmission filter 115 has, as shown in Fig. 9, a band limiting characteristic (high-pass filter characteristic) to transmit a short wavelength or particularly an excited light on a blue short wavelength side and to limit (cut off) transmitted light in the vicinity of a blue long wavelength side or particularly in the vicinity of 450 nm. It is not necessary to transmit the shorter wavelength side than the wavelength region of the excited light, and it may be a band-pass filter characteristic to cut off also the short wavelength side shown by a broken line.

The illumination light emitted from this light source apparatus 105 to the light guide 104a of the light guide cable 104 is incident into the light guide 111 in the endoscope 103, transmitted to the tip end face by this light guide 111 and further made to emit onto the subject side through an illumination lens 117 mounted at an illumination window opposed to this tip end face.

The illumination light emitted onto the subject is partially reflected to become a reflected light and also becomes excitation light to excite a fluorescent substance, and fluorescence is generated by the excitation light. In the third preferred embodiment, a light-sensitive substance is excited by blue excitation light, while fluorescence is generated in a red wavelength region. As shown in Fig. 9, this fluorescence 125 has its intensity peaked at a wavelength of substantially 630 nm.

At the tip end portion of the insertion portion 106, an observation window is provided adjacent to the illumination window, and an objective lens 118 is mounted at the observation window. Also, at an image forming position of this objective lens 118, a tip end face of an image guide 119 is arranged.

An optical image of the subject is formed on the tip end face of this image guide 119, and this optical image is transmitted to a rear end face of the image guide 119. As shown in Fig. 8, the rear end face of this image guide 119 is arranged in the vicinity of the ocular portion 108. Opposite to this rear end face, a cut filter 121 for cutting excitation light is provided through an angle control mechanism 122 for adjusting an angle of the filter surface.

And the optical image transmitted to the rear end face of the image guide 119 is transmitted through the cut filter 121 and an ocular lens 124 mounted at an ocular window of an eye piece 123 rotatably attached to the ocular portion 108. By this, the operator 109 can make observation based on the optical image.

The spectral transmission characteristic of this cut filter 121 is, as shown in Fig. 9, to transmit a red long wavelength side including the wavelength region of fluorescence 125 generated by a fluorescent substance and to cut off in the vicinity of blue on a shorter wavelength side than this red side.

Also, in the wavelength region cut off by this cut filter 121, an overlap portion is formed in which the transmission wavelength region is partially overlapped with that of the excitation light transmission filter 115 provided at the light source apparatus 105. By this overlap portion, a function of illumination light 126 for background is provided for the fluorescence 125. This wavelength has a peak in the vicinity of 450 nm, for example.

And in the third preferred embodiment, as shown in Fig. 8, an angle of the filter surface of the cut filter 121 is made variably adjustable (from an angular state of 90°) by the angle control mechanism 122, provided at the ocular portion 108 so that it is tilted from the state orthogonal to the optical axis (center axis) of the image guide 119.

And by adjusting the angle of the filter surface, a cut-off characteristic by the cut filter 121 formed by an interference filter using interference is made changeable.

That is, by adjusting the angle of the filter surface, the cut-off characteristic can be changed from the state shown by a solid line to the state shown by a dotted line as shown in Fig. 9.

Next, a structure of the angle control mechanism 122 provided at the ocular portion 108 of the endoscope 103 will be described in detail referring to Fig. 8.

The vicinity of the rear end of the image guide 119 is fixed to an exterior member in the ocular portion 108. And opposite to the rear end face of this image guide 119, the cut filter 121 mounted to a filter frame 127 is arranged, and an ocular lens 124 mounted to the eye piece 123 screwed to a threaded portion 128 on the rear side of this cut filter 121 is opposed.

The filter frame 127 is pressed toward the rear side by an elastic force of a ring spring 129, for example, as urging means arranged on a front face of this filter frame 127. On the rear face side of this filter frame 127, a projection portion 130 having a function as a stopper is provided at an upper position, for example, while at a lower position opposing this upper position is arranged a slide member 131 in the state guided by a guide member 132 to be moved forward/backward.

A guide groove, for example, is provided at the guide member 132 in the right and left direction in Fig. 8, and the slide member 131 is fitted in the guide groove. This slide member 131 is in the L-shaped, for example, and its front end is brought into contact with the rear face of the filter frame 127, and the rear face is brought into contact with a front face of the eye piece 123.

And when the eye piece 123 is rotated and this eye piece 123 is moved forward, the slide member 131 is pressed by the front face at that time and moved to the front. Also, with the filter frame 127, the cut filter 121 is inclined from the state parallel with the end face of the image guide 119.

And according to the inclination angle, the cut-off characteristic by the cut filter 121 shown in Fig. 9 is changed.

In the third preferred embodiment with the above construction, the light outputted from the rear end face of the image guide 119 is characterized in that the cut-off characteristic by the cut filter 121 can be shifted in the wavelength axial direction by inclination by the angle control mechanism 122.

And the operator 109 can set a state with an appropriate cut-off characteristic and make fluorescent observation by rotating the eye piece 123 while looking into the ocular lens 124 of the eye piece 123 rotatably attached to the ocular portion 108 by the naked eye.

That is, as shown in Fig. 9, illumination other than the red fluorescent portion is carried out by forming the blue illumination light 126 for background by the overlap portion of the transmission portions of the excitation light transmission filter 115 of the light source apparatus 105 and the cut filter 121 of the endoscope 103.

In this case, if the light intensity of the blue illumination light 126 for background is large, red fluorescence is weakened and hue contrast is deteriorated, while if the intensity is small, the autonomous fluorescence (green to red) of a living tissue by the excitation light becomes dominant, which whitens the tissue and deteriorates the hue contrast.

Thus, it is preferable that an amount of the blue illumination light 126 for background is appropriate, but both the excitation light transmission filter 115 and the cut filter 121 have variations in wavelength tolerances and the light intensity of the illumination light 126 for background was fluctuated by combination of the filters of the light source apparatus 105 and the endoscope 103. In the fluorescent observation, hue plays a very important role in order to check those left behind at detection or ablation of a micro tumor with a hue contrast. For this problem, this preferred embodiment is so constituted that an appropriate hue contrast state can be set easily by the angle control mechanism 122 provided at the ocular portion 108 as above.

Next, operation of the third preferred embodiment will be described referring to Figs. 8 and 9.

By supplying the blue excitation light from the light source apparatus 105 (a part of it is also used for illumination to obtain a reflected image) to the light guide 111 of the endoscope 103, the excitation light transmitted by the light guide 111 is emitted to a living tissue to be observed in a body cavity 102 through the illumination lens 117 at the tip end portion of the insertion portion 106.

An image of the reflected light and fluorescence from the living tissue is formed on the tip end face of the image guide 119 through the objective lens 118, and the optical image is outputted from the rear end face of the image guide 119 to the rear side. This light can be observed by the operator 109 by the naked eye through the ocular lens 124 provided at the eye piece 123 of the ocular portion 108.

At that time, when the operator 109 slides the slide member 131 by rotating the eye piece 123, the inclination angle of the filter surface of the cut filter 121 is changed and the wavelength to be cut off is shifted as shown by the broken line in Fig. 9, which makes brightness (light intensity) of the reflected light changeable.

Since the operator 109 can change the blue hue other than the fluorescence by adjusting increase/decrease of the light intensity of the blue illumination light 126 for background in this way, he or she can set the hue with an appropriate hue contrast against the red fluorescent portion.

Since the operator 109 can solve the hue by wavelength variation only by rotating the eye piece 123, the conventional angle control of the excitation light transmission filter 115 of the light source apparatus 105 is not needed any more.

Also, since the blue hue can be adjusted easily, optimal fluorescence desired by the operator 109 can be created.

Therefore, the third preferred embodiment has the following effect.

With the above construction, even if there is a variation in the overlap portion (cross portion) of the transmission portions by combination of the excitation light transmission filter 115 on the light source apparatus 105 side and the cut filter 121, adjustment can be made to set a state with good operability while observing by the naked eye through the ocular portion 108 without angle control of the excitation light transmission filter 115 on the light source apparatus 105 side.

Also, when a plurality of endoscopes 103 are selectively used for a single light source apparatus 105, an observation state with a hue state with an appropriate hue contrast can be ensured all the time only by initial adjustment when using each of the endoscopes 103.

Also, since adjustment for each operation can be made unnecessary, time for an operation can be saved (reduced).

In the third preferred embodiment, the angle control mechanism 122 for adjusting the cut-off wavelength is formed by rotating the eye piece 123 to adjust the angle of the cut filter 121, but as shown in Fig. 10, an angle control mechanism 122B for adjustment to an appropriate hue contrast state by shifting the cut-off wavelength may be so formed that the angle of the cut filter 121 is adjusted by operating a portion other than the eye piece 123.

Fig. 10 shows a peripheral part of the ocular portion 108 in a variation of the third preferred embodiment. In the angle control mechanism 122B in Fig. 10, a columnar projection portion 135 is projected at one location in the circumferential direction of the filter frame 127 to which the cut filter 121 arranged opposite to the rear end face of the image guide 119 is mounted, a lower end position, for example. And the projection portion 135 is fitted into a recess portion provided on an inner wall surface of an exterior member of the ocular portion 108. Moreover, the filter frame 127 is rotatably held with respect to the exterior member.

Also, a pin 136 is projected at an upper position of the filter frame 127 opposite to the projection portion 135. This pin 136 pierces a hole in the exterior member to be exposed to the outside and is provided with a lug 137. A gap portion required for inclination of the filter frame 127 by a predetermined amount is provided between the filter frame 127 and an inner circumferential face of the exterior member.

And the operator can set an appropriate hue contrast state by adjustment of the angle of the filter surface by rotating this lug 137.

Moreover, an o-ring 138 for water tightness and for generating an appropriate frictional force is provided in the longitudinal direction and around a middle position of this pin 136. Also, an o-ring 139 for water tightness is provided on an outer circumferential face in the vicinity of a front end of the eye piece 123.

In the variation of the third preferred embodiment, the operator 109 can also set an appropriate hue contrast state by adjustment of the angle of the filter surface of the cut filter 121 with good operability by rotating the lug 137 provided at the ocular portion 108 while making observation through the ocular portion 108.

Moreover, the operator 109 can make adjustment of visibility by rotating the eye piece 123 in this case.

### (Fourth embodiment not part of the present invention)

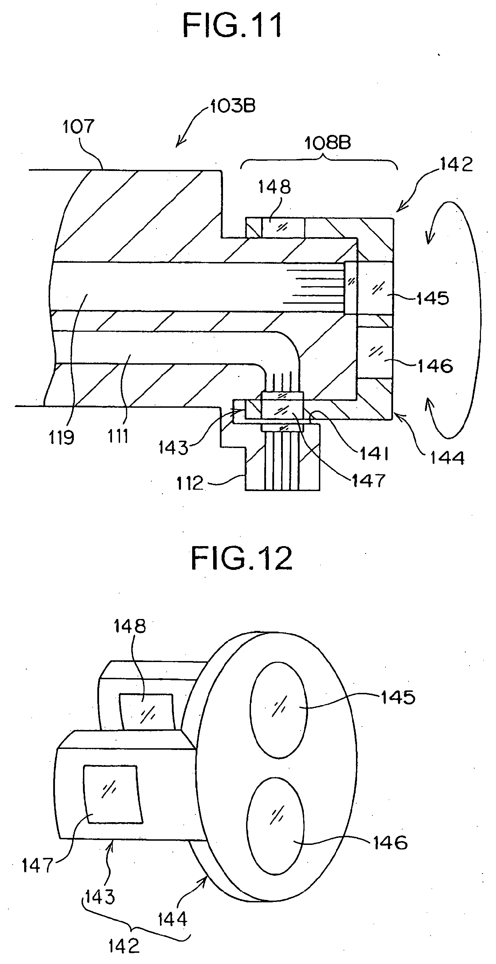

Next, a fourth embodiment will be described referring to Figs. 11 and 12. First, the construction of the fourth embodiment will be described.

The construction of an endoscope system as an endoscope image pickup system in the fourth preferred embodiment is similar to that of the third preferred embodiment, and an endoscope 103B of the fourth preferred embodiment changes the structure of the ocular portion from the endoscope 103 of the third preferred embodiment. Also, a light source apparatus of the fourth preferred embodiment is changed from the excitation light transmission filter 115 of the light source apparatus 105 in Fig. 7 to a filter for ordinary light, though not shown. The structure of the ocular portion of the endoscope 103B in the fourth preferred embodiment will be described below.

As shown in Fig. 11, a base portion 112 of the light guide 111 is provided in the vicinity of an ocular portion 108B in the endoscope 103B of the fourth preferred embodiment. Also, a notch portion 141 is formed in the middle of an L-shaped bent portion in the vicinity of the rear end of this light guide 111 so that a frame portion 143 projecting from a disk surface of an eye piece 142 for fluorescence to form the letter U can be inserted and attached.

This eye piece 142 for fluorescence is provided with a cut filter 145 for cutting excitation light and transmitting fluorescence and a ordinary-light filter 146 for transmitting visible light, that is, ordinary light, at two locations in the circumferential direction in the disk shape eye piece body portion 144 for fluorescence as shown in Fig. 12.

Moreover, a blue filter 147 as a blue excitation light transmission filter for transmitting blue excitation light and a visible light filter 148 for transmitting visible light are provided at the frame portion 143 projecting orthogonally from a plate surface of this eye piece body portion 144 for fluorescence and in the parallel plate shape opposite to each other.

And in the state where the blue filter 147 is inserted into the notch portion 141 and the blue filter 147 is opposed to light guide 111 as shown in Fig. 11, the cut filter 145 is opposed to the rear end face of the image guide 119 so as to make fluorescent observation.

Moreover, when the eye piece 142 for fluorescence is rotated from this state by 180°, the visible light filter 148 is inserted into the notch portion 141, and the ordinary-light filter 146 is set to be opposed to the rear end face of the image guide 119 in this state. And visible-light or ordinary-light observation can be made.

In this way, in the fourth preferred embodiment, fluorescent observation and ordinary-light observation can be switched to each other by rotating the eye piece 142 for fluorescence by 180°.

Both the ordinary-light filter 146 and the visible-light filter 148 can use plain glass not limiting wavelength.

Here, with regard to the filter transmission wavelength, the blue filter 147 has the same characteristics as that of the excitation light transmission filter 115 and the cut filter 145 as that of the cut filter 121 as shown in Fig. 9.

And in the fourth preferred embodiment, in the case of fluorescent observation, both the blue filter 147 transmitting the excitation light and the cut filter 145 are mounted to the single eye piece 142 for fluorescence. Therefore, by selectively attaching either or both of the two filters 47 and 54 at a shipment inspection so that the light intensity of the illumination light 126 for background by the overlap portion shown in Fig. 9 is optimized, when a light-emitting characteristic of the lamp 114 of the light source apparatus 105 is not changed largely, such a state that the combination of the light source apparatus 105 and the endoscope 103B hardly affects can be realized. By this, fluorescent observation can be made with a good hue contrast even without any adjustment in use.

Operation of the fourth preferred embodiment of the above construction will be described. In the case of the ordinary-light observation, the visible-light filter 148 in the eye piece 142 for fluorescence is arranged in the notch portion 141 (that is, in the illumination light transmission path) so that the ordinary light supplied from the light source apparatus 105 is made to transmit through the visible-light filter 148 and to emit to the living tissue side from the tip end face of the light guide 111.

And an optical image formed on the tip end face of the image guide 119 by the light reflected by the living tissue is further transmitted from its rear end face through the ordinary-light filter 146. By this, the operator 109 can observe the optical image by the naked eye.

While in the case of the fluorescent observation, the blue filter 147 is arranged opposite to the light guide 111 in the notch portion 141 as shown in Fig. 11 by rotating the eye piece for fluorescence 142 by 180° and the cut filter 145 is arranged in the opposite manner at the rear end face of the image guide 119.

And in the ordinary light from the light source apparatus 105, only the blue excitation light is transmitted through the blue filter 147 to the light guide 111 and emitted to the living tissue side. And the excitation light reflected by the living tissue and the fluorescence emitted by the living tissue are condensed by the objective lens 118 and an image is formed on the tip end face of the image guide 119.

And this optical image is outputted toward the cut filter 145 side from the rear end face of the image guide 119. By this, the operator 109 can observe the optical image by the naked eye through this cut filter 145.

Since the blue filter 147 and the cut filter 145 are integrally attached to the eye piece 142 for fluorescence of the fourth preferred embodiment in the state where a hue contrast becomes appropriate for the fluorescent observation, the fluorescent observation can be made in the appropriate state hardly affected by the combination of the light source apparatus 105 and the endoscope 103B. Therefore, the fourth preferred embodiment has the following effect.

According to the fourth preferred embodiment, by selecting a combination of two filters with variations in characteristics in advance for a fluorescent observation and using the combination in the fluorescent observation, a variation in the illumination light at the overlap portion can be substantially solved, and angle adjustment of the excitation light transmission filter on the light source apparatus side is not needed any more.

Also, the operator 109 can selectively use a fluorescent image or a visible light image easily only by switching the eye piece 142 for fluorescence.

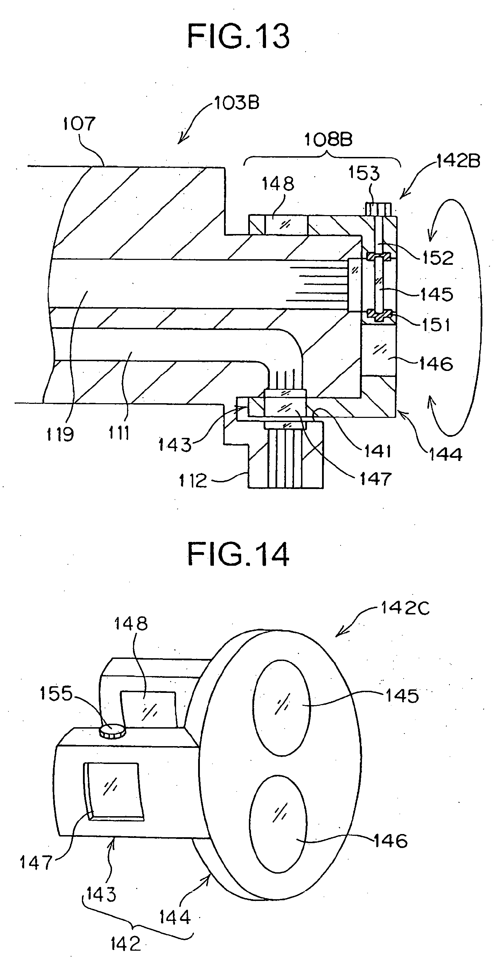

In order that the lamp 114 with different light emitting characteristic can be used as the light source apparatus 105, a structure may be such that an angle of the filter surface of the cut filter 145, for example, can be adjusted as with an eye piece 142B for fluorescence in a first variation of the fourth preferred embodiment shown in Fig. 13.

In the eye piece 142B for fluorescence in Fig. 13, the cut filter 145 is mounted to the filter frame 151 and this filter frame 151 is rotatably mounted on the inside of the observation window of the eyepiece body portion 144 for fluorescence.

Moreover, a lower end of the pin 152 is mounted to this filter frame 151. This pin 152 pierces the hole of the eye piece body portion 144 for fluorescence and is projected outward, and the lug 153 is provided at its upper end. And the operator can tilt the filter surface of the cut filter 145 together with the filter frame 151 by rotatably operating the lug 153 so that the cut-off characteristic can be shifted similarly as in the description for the third preferred embodiment.

By the above construction, the fluorescent observation with an appropriate hue contrast can be made without operating the lug 153 normally.

Also, when the light source apparatus 105 adopts a lamp with light emitting characteristic largely different from an ordinary lamp such as a different type of lamp, the fluorescent observation can be made by setting the state with an appropriate hue contrast by rotating the lug 153.

Moreover, as an eye piece 142C for fluorescence of a second variation of the fourth preferred embodiment schematically shown in Fig. 14, the angle of the filter surface on the blue filter 147 side may be made in a structure capable of adjustment by rotational operation of a lug 155. The structure to tilt the filter surface of the blue filter 147 can be realized by providing the same structure as that shown in Fig. 13.

### (Fifth embodiment not part of the present invention)

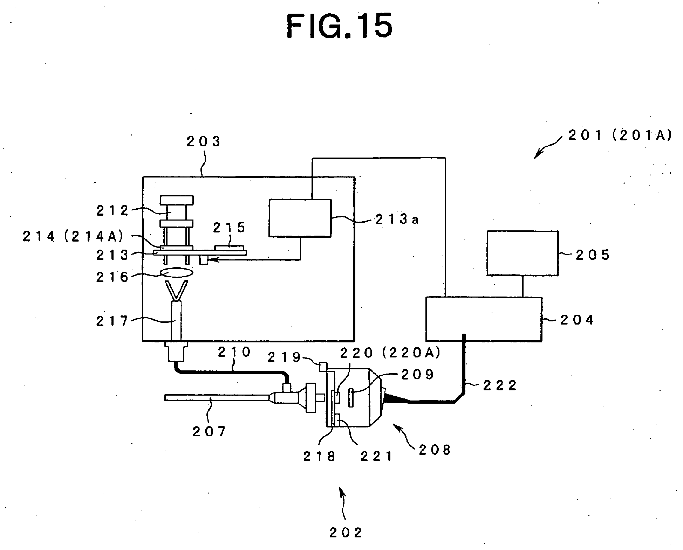

Next, based on Fig. 15, an entire construction of a sentinel lymph node detection system 201 as an endoscope image pickup system according to a fifth embodiment will be described. Fig. 15 is a block diagram for explaining the entire construction of the sentinel lymph node detection system 201 according to the fifth preferred embodiment. As shown in Fig. 15, the sentinel lymph node detection system 201 as the endoscope image pickup system in the fifth preferred embodiment comprises an electronic endoscope 202 provided with image pickup means, a light source apparatus 203 for irradiating a subject with illumination light, TV camera 204 as image processing means for processing an image pickup signal outputted from the electronic endoscope 202 and for generating an image signal, and a monitor 205 as image display means for displaying the image signal outputted from the TV camera 204.

The electronic endoscope 202 is constituted by a rigid scope 207 to be inserted into a body cavity and a camera head portion 208 detachably connected to a base end side of the scope 207. In the camera head portion 208, a CCD 209 as image pickup means is provided for generating an image pickup signal by capturing an image of light from the subject guided by an image guide, not shown, of the scope 207. Also, a light guide cable 210 is extended from a side end face of the scope 207, and its one end is connected to the light source apparatus 203. Moreover, a light guide, not shown, for guiding illumination light transmitted from the light guide cable 210 to the scope 207 to the tip end of the scope 207 is arranged in the electronic endoscope 202.

The light source apparatus 203 has a xenon lamp 212 for generating white light, which is illumination light. The white light emitted from the xenon lamp 212 enters into a rotating plate 213. The rotating plate 213 is provided with a filter 214 for fluorescent observation as first filter means and a filter 215 for ordinary observation, and either of the filters is selected according to an observation mode and inserted onto an optical path of the illumination light by a rotating plate control unit 213a.

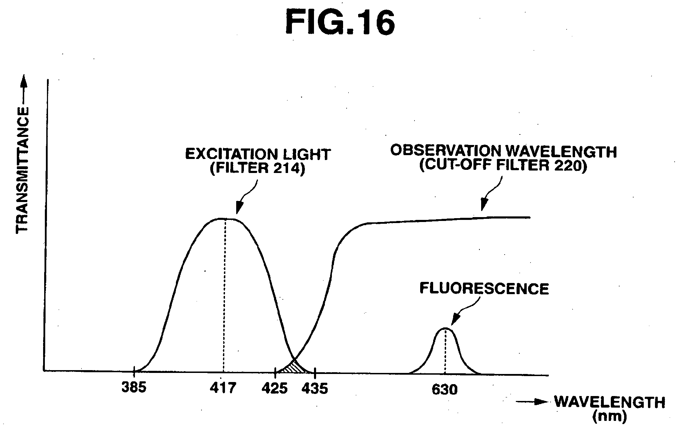

The filter 214 for fluorescent observation is, as shown in Fig. 16, for example, set to transmit a wavelength band of 385 nm to 435 nm with 417 nm as its peak. Fig. 16 is a graph showing an example of transmission characteristics of the filter 214 and a cut-off filter 220 used in the fluorescent observation mode. The transmission characteristic of the filter 214 is set so that it has a peak wavelength according to an absorption maximum wavelength of a fluorescent substance remaining in a tumor when a fluorescent diagnostic agent is administered into a living body and transmits a light of the wavelength region of not less than the width of 15 nm including the peak wavelength. In the fifth preferred embodiment, 5-ALA (5-Aminolevulinic acid) is used as the fluorescent diagnostic agent, and a solution in which 5-ALA is solved in saline is locally injected into the vicinity of the tumor prior to an inspection. 5-ALA injected into the living body is taken into a cell and causes a chemical change to change into PP IX (Protoporphyrin IX), which is a fluorescent substance. PP IX has a such a nature that it is accumulated in a sentinel lymph node, which is a tumor cell, but not accumulated in a normal cell. Also, it has a nature that it emits fluorescence at 630 nm when irradiated with excitation light at 417 nm. Thus, in the fifth preferred embodiment, the filter 214 having the above-mentioned transmission characteristic is used for fluorescent observation. Moreover, the filter 215 for ordinary observation is a filter to transmit the light in the wavelength region of the visible light.

The illumination light with a desired wavelength selectively transmitted according to the observation mode by the rotating plate 213 of the illumination light is condensed by a lens 216 and made to enter into an incident end of a light guide connector 217. The other end of the light guide connector 217 is connected to the light guide cable 210, and the illumination light incident to the light guide connector 217 passes through the light guide cable 210, a light guide, not shown, and an illumination window, not shown, at the tip end face of the light guide scope 207 and is emitted to the subject. In the ordinary observation mode, the visible light is emitted onto the subject, while in the fluorescent observation mode, the excitation light is emitted to the subject.

On the other hand, at the camera head portion 208 of the electronic endoscope 202, the CCD 209 is arranged at a position to condense the light from the subject guided by an image guide, not shown, of the scope 207. A filter frame 218 is arranged at the tip end side of the CCD 209, and either of the cut-off filter 220 as second filter means or an ordinary observation filter 221 is inserted into the filter frame 218 interlockingly with a switching lever 219 provided at the camera head portion 208.

The cut-off filter 220 is inserted into the filter frame 218 in the fluorescent observation mode, and the transmission characteristic of the cut-off filter 220 is set as shown in Fig. 16, for example, to allow light in a wavelength band of not less than 425 nm. The transmission characteristic of the cut-off filter 220 is set to allow to transmit the fluorescence emitted from the fluorescent substance accumulated in the sentinel lymph node but to prevent transmission of the peak wavelength of the excitation light. Also, as the background light, the light on the long wavelength side of the excitation light is set to be transmitted. In the fifth preferred embodiment, since the wavelength of the excitation light is 385 to 435 nm, the light in the wavelength region of 425 to 435 nm in the reflected light from the subject by the excitation light is transmitted by the cut-off filter 220 as the background light. Also, the ordinary observation filter 221 is set to transmit all the light reflected from the subject.

The light condensed at the CCD 209 after being transmitted either by the cut-off filter 220 or the ordinary observation filter 221 inserted into the filter frame 218 is photoelectric-converted by the CCD 209 so as to generate an image pickup signal. The generated image pickup signal passes through the TV camera connector 22 extended from the electronic endoscope 202 to the TV camera 204 and is outputted from the CCD 209 to the TV camera 204. At the TV camera 204, a publicly known signal processing is conducted for the received image pickup signal so as to generate an image signal. The image signal is outputted from the TV camera 204 to the monitor 205, and an endoscopic image illuminated and captured by ordinary white light or a fluorescent image illuminated and captured by excitation light is displayed.

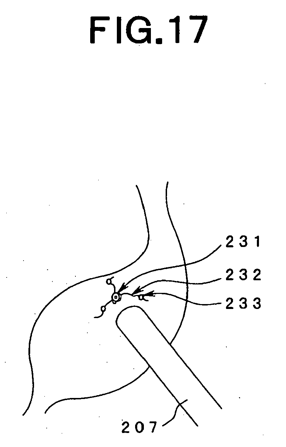

Operation of the sentinel lymph node detection system 201 constructed as above will be described. First, the operation when the fluorescent observation mode was selected will be described. Prior to an inspection, a solution in which 5-ALA is solved in saline is locally injected into the vicinity of a tumor 231 of a subject, which is a portion to be inspected. 5-ALA causes a chemical change and changes to PP IX, which is a fluorescent substance, passes through a lymphatic vessel 232 and is accumulated in a sentinel lymph node 233. When the inspection is started, as shown in Fig. 17, the scope 207 is arranged at a position opposed to the subject. Fig. 17 is a schematic view for explaining arrangement of the scope 207 at the inspection. Next, when power, not shown, of the sentinel lymph node detection system 201 is turned on, white light, which is illumination light, is emitted from the xenon lamp 212 of the light source apparatus 203 and is incident on the rotating plate 213.

When the fluorescent observation mode was selected, the filter 214 was inserted onto the optical path of the illumination light. Thus, the incident illumination light is transmitted by the filter 214 and only the excitation light in the wavelength band of 385 to 435 nm is filtered and sequentially emitted from the light source apparatus 203 to the light guide cable 210. The excitation light having entered into the light guide cable 210 passes through the illumination window, not shown, at the tip end face of the scope 207 and is emitted to the subject. By illumination of the excitation light, fluorescence with the wavelength of 630 nm is generated from the sentinel lymph node 233 of the subject. Also, scattered light, reflected light and radiated light are generated from a normal tissue of the subject.

These lights pass through the image guide, not shown, of the scope 207 and are guided to the camera head 208. When the fluorescent observation mode was selected, the cut-off filter 220 is arranged at the filter frame 218, and the light guided to the camera head portion 208 is transmitted by the cut-off filter 220, and the light of the wavelength region less than 425 nm is cut off. That is, the fluorescence of 630 nm emitted from the sentinel lymph node 233 of the subject and the light with the wavelength of 425 to 435 nm (background light) are transmitted among the scattered light, reflected light and radiated light from the normal tissue of the subject, an image is formed on an electric conversion surface of the CCD 209 and electric-converted. The image pickup signal electric-converted and outputted from the CCD 209 is inputted to the TV camera 204, and a publicly known signal processing is carried out to generate an image signal, which is outputted to the monitor 205. On the monitor 205, a fluorescent image made of a fluorescent image of the sentinel lymph node 233 by the fluorescence at the wavelength of 630 nm and an image of the subject by the background light at the wavelength of 425 to 435 nm is displayed.

Next, operation of the ordinary observation mode will be described. The operator operates the switching lever 219 provided at the camera head portion 208 and inserts the ordinary observation filter 221 into the filter frame 218. Also, the operator operates the rotating plate control unit 213a and rotates the rotating plate 213 so that the filter 215 for ordinary observation is inserted onto the optical path of the illumination light. Then, the white light emitted from the xenon lamp 212 of the light source apparatus 203 and entering into the rotating plate 213 is transmitted by the filter 215 and the light in the wavelength band of the visible light is filtered and sequentially emitted from the light source apparatus 203 to the light guide cable 210. The excitation light having entered into the light guide cable 210 passes through the illumination window, not shown, at the tip end face of the scope 207 and emitted onto the subject.

By illumination of the illumination light, scattered light, reflected light and radiated light are generated from the subject. These lights are guided through an image guide, not shown, of the scope 207 to the camera head portion 208 and transmitted by the ordinary observation filter 221 and then, an image is formed on a photoelectric conversion surface of the CCD 209 and photoelectric-converted. Here, the ordinary observation filter 221 is set to transmit all the lights from the subject. An image pickup signal photoelectric-converted and outputted from the CCD 209 is inputted to the TV camera 204, given a publicly known signal processing so as to generate an image signal and outputted to the monitor 205. The monitor 205 displays an endoscopic image illuminated and captured by ordinary visible light.

In this way, the sentinel lymph node detection system 201 of this preferred embodiment is set so that the wavelength band of the excitation light and the wavelength band of the fluorescence do not overlap each other, and the fluorescence emitted from the sentinel lymph node can be easily separated from the reflected light from the peripheral normal tissue. Thus, detection sensitivity of the fluorescence can be improved and a sentinel lymph node can be detected in a short time with a high accuracy. Also, as shown in Fig. 16, since the extended portion on the long wavelength side of the transmission characteristic of the filter 214 is set to be overlapped with the extended portion of the short wavelength side of the transmission characteristic of the cut-off filter 220, the excitation light in the wavelength band corresponding to the overlap portion can be used as background light, and there is no need to separately provide a filter for generating background light other than the filter 214, which promotes simplification of the system construction and reduction of costs.

### (Sixth embodiment not part of the present invetion)

Next, a sixth embodiment will be described. A sentinel lymph node detection system 201A as an endoscope image pickup system in the sixth preferred embodiment has the same construction as that of the fifth preferred embodiment as shown in Fig. 15 except that the transmission characteristic of the filter 214A and the transmission characteristic of the cut-off filter 220A are different from the transmission characteristics of the filter 214 and the cut-off filter 220, respectively. Then, the same reference numerals are given to the same construction and the description will be omitted. Only the transmission characteristics of the filter 214A and the cut-off filter 220A characterizing this preferred embodiment will be described below.

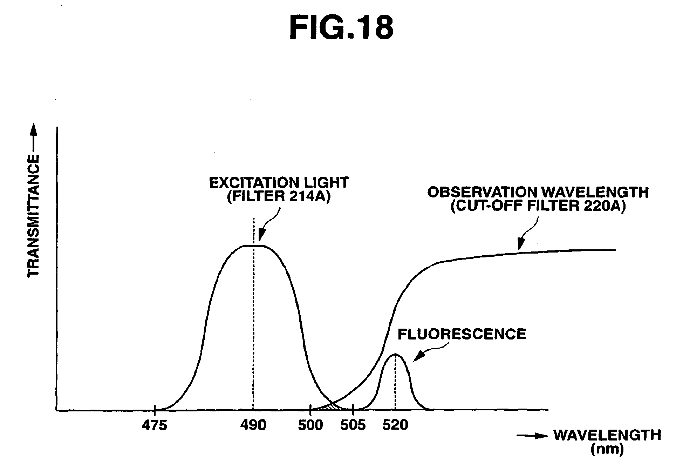

The filter 214A for fluorescent observation in the sixth preferred embodiment is set, as shown in Fig. 18, for example, to transmit the wavelength band of 475 to 505 nm with a peak at 490 nm. Fig. 18 shows a graph for explaining an example of the transmission characteristics of the filter 214A and the cut-off filter 220A used in the fluorescent observation mode. The transmission characteristic of the filter 214A is set so that it has a peak wavelength according to the absorption maximum wavelength of the fluorescent substance remaining in the tumor and the light of the wavelength band of the width of not less than 15 nm including the peak wavelength is transmitted when a fluorescent diagnostic agent is administered into a living body.

In the sixth preferred embodiment, FITC (Fluorescsein-Isothiocyanate) is used as the fluorescent diagnostic agent, and a solution in which FITC is solved in saline is locally injected into the vicinity of a tumor prior to an inspection. FITC injected into a living body has a nature as with PP IX that it is accumulated in a sentinel lymph node, which is a tumor cell, but not accumulated in a normal cell. Also, it has a nature that it emits fluorescence at 520 nm when irradiated with excitation light at 490 nm. Thus, in this preferred embodiment, the filter 214A having the above-mentioned transmission characteristic is used for fluorescent observation.

The cut-off filter 220A is set, as shown in Fig. 18, for example, to transmit a light in the wavelength band of not less than 500 nm. The transmission characteristic of the cut-off filter 220A is set to transmit fluorescence emitted from a fluorescent substance accumulated in a sentinel lymph node but not the peak wavelength of the excitation light. Also, it is set so that the light on the long wavelength side of the excitation light is transmitted as background light. In this preferred embodiment, since the wavelength of the excitation light is 475 to 505 nm, the light in the wavelength band of 500 to 505 nm in the reflected light from the subject by the excitation light is transmitted by the cut-off filter 220A as the background light.

Since operation of the sentinel lymph node detection system 201A constructed as above is the same as that of the fifth preferred embodiment, the description will be omitted.

In this way, since the lymph node detection system 201A of the sixth preferred embodiment is set so that the wavelength band of the excitation light and the wavelength band of fluorescence do not overlap each other and the fluorescence emitted from the sentinel lymph node can be easily separated from the reflected light from the peripheral normal tissue as with the fifth preferred embodiment, detection sensitivity of fluorescence can be improved and a sentinel lymph node can be detected in a short time and with a high accuracy. Moreover, the excitation light in the wavelength band corresponding to the characteristic overlap portion of the filter 214A and the cut-off filter 220A can be used as the background light, and there is no need to separately provide a filter for generating background light other than the filter 214A, which promotes simplification of the system construction and reduction of costs.

The fluorescent diagnostic agent used for the inspection is not limited to the above-mentioned 5-ALA or FITC but may be those including or generating a fluorescent substance having such a characteristic that the wavelength band of the excitation light and the wavelength band of the fluorescence do not overlap with each other. And fluorescent substances such as Texas Red with the absorption maximum wavelength of 596 nm and the fluorescent maximum wavelength of 620 nm or TRITC (Tetramethylrhodamine-Isothiocyanate) with the absorption maximum wavelength of 541 nm and the fluorescent maximum wavelength of 572 nm may be used as a fluorescent diagnostic agent. In this case, the transmission characteristics of the filter 214 for fluorescent observation and the cut-off filter 220 are set according to the characteristic of a fluorescent substance to be used.

## Claims

1. An endoscope image pickup system (1) comprising:
light source means (2) for generating light in a visible light wavelength region including blue excitation light;
an excitation light transmission filter (11a) provided at the light source means (2) and transmitting the blue excitation light;
an ordinary light filter (11b) provided at the light source means (2) for transmitting light in the visible light wavelength region;
an endoscope (3) for irradiating light from the light source means (2) so as to obtain an optical subject image using light reflected by a subject;
an endoscopic filter (28) provided at the endoscope (3) for transmitting only a part of the light in the visible light wavelength region and of the excitation light and transmitting fluorescence;
image pickup means (4) attached to an ocular portion of the endoscope for picking up an image of the subject according to each of the lights transmitted by the endoscopic filter; and
image processing means (5) for processing an image pickup signal from the image pickup means for image formation,
wherein the excitation light transmission filter (11a) and the ordinary light filter (11b) are switchable such that the excitation light transmission filter (11a) is arranged on the optical path of the light source means (2) when the system (1) is in a fluorescent observation mode and the ordinary light filter (11b) is arranged on the optical path of the light source means (2) when the system (1) is in an ordinary observation mode,
wherein the endoscope image pickup system (1) includes:
composite image generating means (53) provided at the image processing means (5), and
color tone correcting means (52),
wherein the color tone correcting means is adapted to obtain a gain value for adjusting a blue color signal outputted from a signal processing circuit connected to the image pickup means to a constant value when an image of an inner surface of a B balance cap irradiated with the blue excitation light is picked up, and correct a luminance level of a blue excitation light image obtained by picking up an image of the subject by using the obtained gain value, and the composite image generating means (53) is adapted to generate a composite image by combining a fluorescent image obtained by picking up an image of the subject and a blue excitation light image corrected by the color tone correcting means (52), when the system (1) is in the fluorescent observation mode, and
the composite image generating means (53) is adapted to generate a composite image from different color signals (R, G, B) corresponding to component images obtained using the light in the visible light wavelength region and the color tone correcting means (52) is adapted to amplify the color signal corresponding to the wavelength of the blue excitation light, when the system (1) is in the ordinary observation mode.

2. The endoscope image pickup system (1) according to claim 1, wherein the endoscopic filter (28) has a filter characteristic for transmitting fluorescence in a red wavelength region generated by a light-sensitive substance by the blue excitation light.

3. The endoscope image pickup system (1) according to claim 1, wherein the excitation light transmission filter (11a) is a double band-pass filter having a transmission wavelength of the blue excitation light and illumination light for background not overlapping the blue excitation light and set within a transmission wavelength region transmitted by the endoscopic filter.

## Patentansprüche

1. Endoskopisches Bildaufnahmesystem (1), aufweisend:
ein Lichtquellenmittel (2) zum Erzeugen von Licht im sichtbaren Wellenlängenbereich einschließlich blauen Erregungslichtes;
ein am Lichtquellenmittel (2) vorgesehenes Anregungslicht-Transmissionsfilter (11a), das das blaue Anregungslicht durchlässt;
ein am Lichtquellenmittel (2) vorgesehenes normales Lichtfilter (11b), das das Licht im sichtbaren Wellenlängenbereich durchlässt;
ein Endoskop (3) zum Abstrahlen des Lichtes vom Lichtquellenmittel (2), um ein optisches Bild des Objekts mittels des von einem Objekt reflektierten Lichtes zu erhalten;
ein Endoskop-Filter (28) am Endoskop (3), das nur einen Teil des Lichtes im sichtbaren Wellenlängenbereich und des Anregungslichtes durchlässt und das Fluoreszenz durchlässt;
ein Bildaufnahmemittel (4), das an einem Okularabschnitt des Endoskops angebracht ist, um ein Bild des Objekts entsprechend jeder Lichtart aufzunehmen, die vom Endoskop-Filter (28) durchgelassen wird; und
ein Bildverarbeitungsmittel (5) zum Verarbeiten eines Bildaufnahmesignals vom Bildaufnahmemittel zur Bildgebung,
wobei das Anregungslicht-Transmissionsfilter (11a) und das normale Lichtfilter (11b) umschaltbar sind, so dass das Anregungslicht-Transmissionsfilter (11a) in einem optischen Pfad des Lichtquellenmittels (2) angeordnet ist, wenn sich das System (1) im Fluoreszenz-Beobachtungsmodus befindet und das normale Lichtfilter (11b) im optischen Pfad des Lichtquellenmittels (2) angeordnet ist, wenn sich das System (1) im normalen Beobachtungsmodus befindet,
wobei das endoskopische Bildaufnahmesystem (1) enthält:
ein Generatormittel (53) für ein zusammengesetztes Bild, das am Bildverarbeitungsmittel (5) vorgesehen ist, und
ein Farbton-Korrekturmittel (52),
wobei das Farbton-Korrekturmittel dazu eingerichtet ist, einen Verstärkungswert zum Einstellen eines von einer mit dem Bildaufnahmemittel verbundenen Signalverarbeitungsschaltung ausgegebenen blauen Farbsignals auf einen konstanten Wert zu gewinnen, wenn ein Bild der Innenoberfläche einer mit dem blauen Anregungslicht bestrahlten B-Abstimmkappe aufgenommen wird, und einen Leuchtstärkepegel des Bildes mit blauem Anregungslicht, der durch Aufnehmen eines Bildes des Objekts mittels des gewonnenen Verstärkungswertes erhalten wird, zu korrigieren, und das Generatormittel (53) für ein zusammengesetztes Bild dazu eingerichtet ist, ein zusammengesetztes Bild zu erzeugen, indem es ein fluoreszierendes Bild, das durch Aufnehmen eines Bildes des Objekts erhalten wird, und ein mit dem Farbton-Korrekturmittel (52) korrigiertes Bild mit blauem Anregungslicht kombiniert, wenn sich das System (1) im Fluoreszenz-Beobachtungsmodus befindet, und
das Generatormittel (53) für ein zusammengesetztes Bild dazu eingerichtet ist, ein zusammengesetztes Bild aus unterschiedlichen Farbsignalen (R, G, B) entsprechend den Komponentenbildern zu erzeugen, die mit dem Licht im sichtbaren Wellenlängenbereich erhalten werden, und das Farbton-Korrekturmittel (52) dazu eingerichtet ist, das Farbsignal entsprechend dem blauen Anregungslicht zu verstärken, wenn sich das System (1) im normalen Beobachtungsmodus befindet.

2. Endoskopisches Bildaufnahmesystem (1) nach Anspruch 1, wobei das Endoskopfilter (28) eine Filtercharakteristik hat, die von einer lichtempfindlichen Substanz durch das blaue Anregungslicht erzeugte Fluoreszenz durchlässt.

3. Endoskopisches Bildaufnahmesystem (1) nach Anspruch 1, wobei das Anregungslicht-Transmissionsfilter (11a) ein Doppel-Bandpassfilter mit einer Transmissionswellenlänge für das blaue Anregungslicht ist, und das Beleuchtungslicht für den Hintergrund das blaue Anregungslicht nicht überlappt und innerhalb eines Transmissionswellenlängenbereichs, der vom Endoskopfilter durchgelassen wird, eingestellt ist.

## Revendications

1. Système (1) de capture d'image endoscopique comprenant :
un moyen (2) de source de lumière destiné à générer une lumière dans une région de longueur d'onde de lumière visible comprenant une lumière d'excitation bleue ;
un filtre (11a) de transmission de lumière d'excitation prévu au niveau du moyen (2) de source de lumière et transmettant la lumière d'excitation bleue ;
un filtre (11b) de lumière ordinaire prévu au niveau du moyen (2) de source de lumière pour transmettre une lumière dans la région de longueur d'onde de lumière visible ;
un endoscope (3) destiné à irradier une lumière à partir du moyen (2) de source de lumière de façon à obtenir une image optique d'un sujet en utilisant la lumière réfléchie par un sujet ;
un filtre endoscopique (28) prévu au niveau de l'endoscope (3) pour transmettre uniquement une partie de la lumière dans la région de longueur d'onde de lumière visible et de la lumière d'excitation et transmettre une fluorescence ;
un moyen (4) de capture d'image fixé à une partie oculaire de l'endoscope pour capturer une image du sujet conformément à chacune des lumières transmises par le filtre endoscopique ; et
un moyen (5) de traitement d'image destiné à traiter un signal de capture d'image provenant du moyen de capture d'image pour la formation d'image,
dans lequel le filtre (11a) de transmission de lumière d'excitation et le filtre (11b) de lumière ordinaire peuvent être commutés d'une manière telle que le filtre (11a) de transmission de lumière d'excitation est agencé sur le trajet optique du moyen (2) de source de lumière lorsque le système (1) se trouve dans un mode d'observation par fluorescence et le filtre (11b) de lumière ordinaire est agencé sur le trajet optique du moyen (2) de source de lumière lorsque le système (1) se trouve dans un mode d'observation ordinaire,
dans lequel le système (1) de capture d'image endoscopique comprend :
un moyen (53) de génération d'image composite prévu au niveau du moyen (5) de traitement d'image, et
un moyen (52) de correction de tonalité de couleur,
dans lequel le moyen de correction de tonalité de couleur est adapté pour obtenir une valeur de gain pour ajuster un signal de couleur bleue délivré en sortie depuis un circuit de traitement de signal connecté au moyen de capture d'image à une valeur constante lorsqu'une image d'une surface interne d'un condensateur de balance des bleus irradiée avec la lumière d'excitation bleue est capturée, et corriger un niveau de luminance d'une image de lumière d'excitation bleue obtenue en capturant une image du sujet en utilisant la valeur de gain obtenue, et le moyen (53) de génération d'image composite est adapté pour générer une image composite en combinant une image de fluorescence obtenue en capturant une image du sujet et une image à la lumière d'excitation bleue corrigée par le moyen (52) de correction de tonalité de couleur, lorsque le système (1) se trouve dans le mode d'observation par fluorescence, et
le moyen (53) de génération d'image composite est adapté pour générer une image composite à partir de différents signaux de couleur (R, V, B) correspondant à des images constitutives obtenues en utilisant la lumière dans la région de longueur d'onde de lumière visible et le moyen (52) de correction de tonalité de couleur est adapté pour amplifier le signal de couleur correspondant à la longueur d'onde de la lumière d'excitation bleue, lorsque le système (1) se trouve dans le mode d'observation ordinaire.

2. Système (1) de capture d'image endoscopique selon la revendication 1, dans lequel le filtre endoscopique (28) possède une caractéristique de filtre destinée à transmettre la fluorescence dans une région de longueur d'onde de rouge générée par une substance photosensible par la lumière d'excitation bleue.

3. Système (1) de capture d'image endoscopique selon la revendication 1, dans lequel le filtre (11a) de transmission de lumière d'excitation est un filtre double passe-bande ayant une longueur d'onde de transmission de la lumière d'excitation bleue et la lumière d'éclairage pour l'arrière-plan ne chevauchant pas la lumière d'excitation bleue et établi à l'intérieur d'une région de longueur d'onde de transmission transmise par le filtre endoscopique.
